(19) Europäisches Patentamt
European Patent Office
Office européen des brevets

(11) Publication number: **0 623 282 A1**

(12) **EUROPEAN PATENT APPLICATION**

(21) Application number: **94201225.3**

(22) Date of filing: **02.05.94**

(30) Priority: **05.05.93 EP 93303475**

(43) Date of publication of application: **09.11.94 Bulletin 94/45**

(84) Designated Contracting States: **AT BE CH DE DK ES FR GB GR IE IT LI LU MC NL PT SE**

(51) Int. Cl.5: **A01N 43/80**, C07D 275/03

(71) Applicant: **SHELL INTERNATIONALE RESEARCH MAATSCHAPPIJ B.V.**
**Carel van Bylandtlaan 30**
**NL-2596 HR Den Haag (NL)**

(72) Inventor: **Davis, Royston Henry**
**76 Bell Road**
**Sittingbourne, Kent ME10 4HE (GB)**
Inventor: **Krummel, Gunther**
**In den Neun Morgen 10**
**D-55127 Mainz (DE)**

(74) Representative: **Allam, Peter Clerk et al**
**LLOYD WISE, TREGEAR & CO.**
**Norman House**
**105-109 Strand**
**London WC2R 0AE (GB)**

(54) **Pesticidal methods and compounds.**

(57) The invention provides a method of combating caterpillar pests, using a compound of general formula

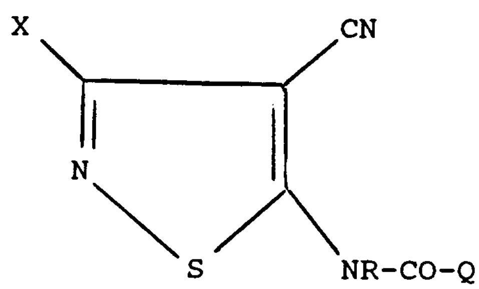

I

where X represents a halogen atom, R represents a hydrogen atom or an optionally substituted alkyl group, and Q represents an optionally substituted aryl or heteroaryl group.

Certain compounds of general formula I are novel.

EP 0 623 282 A1

The present invention relates to the combating of certain insect pests using isothiazole compounds, some of which are novel. Aspects of the invention relate to the use of such compounds in combating larval pests, to novel compounds, compositions and synthetic processes.

U.S. Patent No. 3541108 discloses pesticidal isothiazole derivatives of the formula

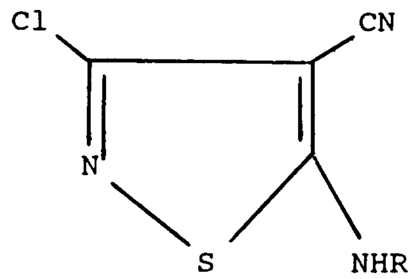

wherein R may be $COR_1$ where $R_1$ is an alkyl radical containing 1 to 10 carbon atoms or phenyl.

Insecticidal and herbicidal activity of the compounds of U.S. Patent No. 3541108 is disclosed, the former being against Musca domestica (housefly) and Blatta orientalis (oriental cockroach).

G.B. Patent No. 1226913 discloses a method for the preparation of pesticidal, especially herbicidal and insecticidal, compounds of general formula

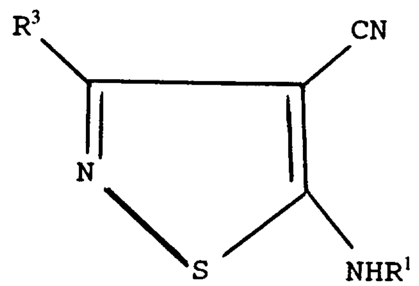

I

in which $R^1$ is a hydrogen atom or an alkyl radical containing up to 6 carbon atoms or is a $COR^2$ radical, $R^2$ is Ar or a straight or branched chain alkyl or alkenyl radical containing up to 18 carbon atoms, which can be substituted by at least one halogen atom, nitrile group or radical Ar and/or in which one or more $-CH_2-$ groups can be replaced by oxygen and/or an -NH- group which may be substituted and/or CO, and/or in which one or more =CH-groups are replaced by nitrogen, or $R^2$ is a cycloalkyl radical containing 3-6 carbon atoms, or is a furyl, pyrrolyl or thienyl radical which can be substituted by at least one halogen atom, nitro or sulphonic acid group or acetyl radical, Ar is a phenyl or naphthyl radical which can be substituted by at least one halogen atom, cyano, nitro, hydroxyl, mercapto or amino group or alkyl, alkoxy, alkylthio, alkylamino or dialkylamino radicals, wherein the alkyl radicals or parts of radicals contain up to 4 carbon atoms, and $R^3$ is a chlorine or bromine atom.

It has now been determined that certain isothiazole compounds, some of which are novel, show interesting and unexpected activity in combating larval pests of lepidoptera species.

According to one aspect of the present invention there is provided a method of combating caterpillar pests at a locus, which comprises treating the locus with a compound of general formula

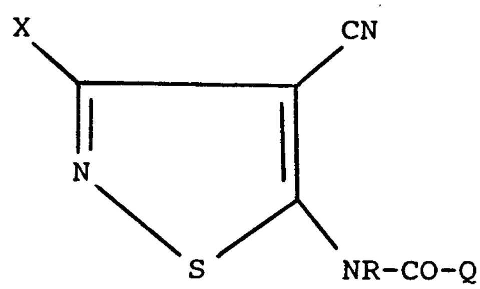

where X represents a halogen atom, R represents a hydrogen atom or an optionally substituted alkyl group, and Q represents an optionally substituted aryl or heteroaryl group.

Unless otherwise stated in this specification, an alkyl group may be linear or branched and suitably contains up to 10, preferably up to 6, and most preferably up to 4, carbon atoms, preferred examples being methyl and ethyl.

Unless otherwise stated in this specification, when any groups are designated as being optionally substituted, the substituent groups which are optionally present may be any of those customarily employed in the development of pesticidal compounds, and/or the modification of such compounds to influence their structure/activity, persistence, penetration or other property. In relation to a halogen atom, this may suitably denote iodine or, especially, bromine, fluorine or chlorine. In general chlorine is a preferred halogen atom. In relation to an alkyl group, specific examples of such substituents may include halogen atoms, and phenyl, alkoxy, alkylthio, hydroxy and cyano groups. In relation to an aryl or heteroaryl moiety, optional substituents may include halogen atoms, and nitro, cyano, alkoxy, haloalkoxy, hydroxy, amino, alkylamino, alkyl and haloalkyl (especially $CF_3$) groups. There may suitably be 0-3 substituents.

A preferred halogen atom X is a chlorine atom.

Preferably R represents a hydrogen atom or a substituted methyl group, suitably a benzyl or a ($C_{1-4}$alkoxy)methyl group. However, most preferably R represents a hydrogen atom.

A suitable optionally substituted heteroaryl group Q may include an optionally substituted thienyl group and an optionally substituted pyridyl group. Preferably a substituted heteroaryl group has a single substituent. A preferred substituent is a halogen atom.

A preferred optionally substituted aryl group is an optionally substituted phenyl group. Preferably this may have 1, 2 or 3 substituents.

Preferably, Q as a phenyl group has a substituent at the 3-position, and optionally one further substituent, at the 4- or, most preferably, the 5- position, or two further substituents, at the 4- and the 5-positions.

Preferred optional substituent(s) of a phenyl group Q include haloalkyl, alkyl, alkoxy, nitro and cyano group(s) and, most preferably, halogen atom(s).

Particularly preferred groups Q include phenyl groups having a halogen substituent at the 3-position, for example 3,4-dihalophenyl, 3,5-dihalophenyl groups and, especially, 3-halophenyl groups. Such compounds of general formula I, which show particularly interesting activity may be regarded as a novel selected class of compounds and accordingly constitute a further aspect of the present invention. A particularly preferred compound is 3-chloro-4-cyano-5-(3-chlorobenzoylamino) isothiazole.

A further novel class of compounds is constituted by compounds of the general formula I in which R represents an optionally substituted alkyl group and accordingly such compounds provide a further aspect of the present invention.

In accordance with a further aspect of the present invention there is provided a process for the preparation of a novel compound of the general formula I, which process comprises reacting together compounds of general formula II and III

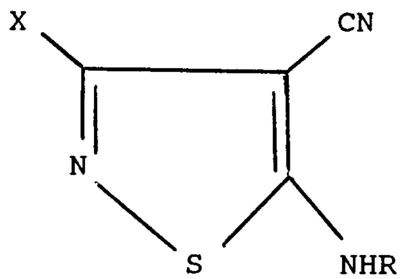

II

Hal-CO-Q III

The reaction may suitably be effected under standard acylation conditions, in the presence of an organic solvent. Examples include pyridine, dimethylformamide and acetonitrile. A basic solvent may be employed, and/or an additional base, for example an alkali metal base, an example being potassium carbonate. The reaction may suitably be effected at an elevated temperature, for example from 50 °C to the reflux temperature.

When the process is applied to prepare a novel compound of general formula I in which R represents an optionally substituted alkyl group, the required compound of general formula II may suitably be prepared via the corresponding 5-amino isothiazole, reacted with a compound R-Hal, suitably in the presence of an organic solvent, for example acetonitrile, and an alkali metal base, for example potassium carbonate, suitably at an elevated temperature; or via the corresponding 5-halo isothiazole, reacted with a compound $RNH_2$, suitably in the presence of an organic solvent, for example tetrahydrofuran, suitably at ambient temperature. Both such methods are described hereafter in greater detail.

The term Hal preferaby denotes a chlorine atom.

The invention also provides a pesticidal composition comprising a carrier and, as active ingredient, a novel compound of general formula I.

The invention further provides the use of a compound of general formula II, in combating larvae of lepidoptera species.

The target pests may be the larval "caterpillar" or "worm" forms of insects of the genus Heliothis, such as H.zea (corn earworm), cotton bollworm, tomato fruitworm, and H. virescens (tobacco budworm); the genus Agrotis, such as A. insilon (black cutworm); the genus Trichoplusia, such as T.ni (cabbage looper); the genus Plutella, such as Plutella xylostella (diamond back moth); and the genus Spodoptera, such as S. littoralis (Egyptian cotton leafworm). A preferred aspect of the present invention therefore relates to the pesticidal treatment of such pests.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus, preferably at least one carrier in a composition

according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are ususally formulated as a dust concentrate having a similar composition to that of a wettable powder but without a dispersant, and are diluted in the field with further solid carrier to give a composition usually containing $^1/_2$-10% w of active ingredient. Granules are usually prepared to have a size between 10 and 100 BS mesh (1.676 - 0.152 mm), and may be manufactured by agglomeration or impregnation techniques. Generally, granules will contain $^1/_2$-75% w active ingredient and 0-10% w of additives such as stabilisers, surfactants, slow release modifiers and binding agents. The so-called "dry flowable powders" consist of relatively small granules having a relatively higher concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are ususally compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick "mayonnaise"-like consistency.

Compositions in accordance with the invention may also contain other ingredients, for example other compounds possessing pesticidal, herbicidal, or fungicidal properties. The compounds of the invention may be found to be especially useful when applied in admixture with insecticides and/or acaricides, e.g. organophosphates, pyrethroids, ureas and organotin compounds, for example the commercial products fenvalerate, permethrin, cypermethrin, deltamethrin, alphacypermethrin, fenbutatin oxide, flufenoxuron diflubenzuron and trefluron.

The invention will be further understood from the following illustrative examples.

Example 1

Preparation of 3-chloro-4-cyano-5-(3-chlorobenzoylamino) isothiazole

[X = Cl; R = H; Q = 3-Cl-Ph]

3-Chloro-4-cyano-5-amino isothiazole (0.8g) was stirred in acetonitrile (50ml), together with potassium carbonate (0.7g). 3-Chlorobenzoyl chloride was added and the reaction mixture was heated to 100 °C, for 3 hours. The solvent was removed by evaporation and the residue titurated in water, and acidified to a pH of about 1, using 2M hydrochloric acid. The solid was filtered and recrystallised from acetone-methanol (1/1, v/v) to give the title compound as a cream solid (mp 260 °C; 1.20g).

| Analysis | | | |
|---|---|---|---|
| | %C | %H | %N |
| Calc. | 44.3 | 1.7 | 14.1 |
| Found | 44.5 | 2.1 | 14.4 |

Examples 2-31

Further compounds as noted in Table 1 were prepared by processes analogous to the process of Example 1. Characterising data for the compounds of Examples 2-31 is noted in Table 2, where available.

**TABLE 1**

| Ex. No. | X | R | Q |
|---|---|---|---|
| 2 | Cl | H | 2-Cl-Ph |
| 3 | Cl | H | 2-F-Ph |
| 4 | Cl | H | 4-$CF_3$-Ph |
| 5 | Cl | H | 4-Cl-Ph |
| 6 | Cl | H | 3-$NO_2$-Ph |
| 7 | Cl | H | 3-F-Ph |
| 8 | Cl | H | 3-$CF_3$-Ph |
| 9 | Cl | H | 3-Br-Ph |
| 10 | Cl | H | 3-$CH_3$-Ph |
| 11 | Cl | H | 3-$OCH_3$-Ph |
| 12 | Cl | H | 3-CN-Ph |
| 13 | Cl | H | 3,4-$Cl_2$-Ph |
| 14 | Cl | H | 3,4-$F_2$-Ph |
| 15 | Cl | H | 3-Cl,4-F-Ph |
| 16 | Cl | H | 3-Br,4-F-Ph |
| 17 | Cl | H | 3,5-$Cl_2$-Ph |
| 18 | Cl | H | 3,5-$F_2$-Ph |
| 19 | Cl | H | 3,5-$Br_2$-Ph |
| 20 | Cl | -$CH_2$-Ph | 3-Cl-Ph |
| 21 | Cl | -$CH_2$-O-$C_2H_5$ | 3-Cl-Ph |
| 22 | Cl | H | Ph |
| 23 | Cl | H | 4-F-Ph |
| 24 | Cl | H | 6-Cl-pyrid-3-yl |
| 25 | Cl | H | 5-Cl-thien-2-yl |
| 26 | Cl | H | 2,4,5-$F_3$-Ph |
| 27 | Cl | H | 3,4,5-$F_3$-Ph |
| 28 | Cl | H | 3-$NO_2$,4-Cl-Ph |

| Ex. No. | X | R | Q |
|---|---|---|---|
| 29 | Cl | H | 3-$NO_2$,4-F-Ph |
| 30 | Cl | $-CH_2-O-CH_3$ | 3-Cl-Ph |
| 31 | Cl | $-CH_2-O-CH_3$ | 3-Br,4-F-Ph |

**TABLE 2**

| EXAMPLE NO. | ANALYSIS | | | MP/°C |
|---|---|---|---|---|
| | %C | %H | %N | |
| 2 | calc | | | 213-215 |
| | found | | | |
| 3 | calc | | | 128-131 |
| | found | | | |
| 4 | calc 43.4 | 1.5 | 12.7 | 300 |
| | found 43.6 | 2.0 | 12.5 | |
| 5 | calc | | | 298-300 |
| | found | | | |
| 6 | calc 42.7 | 1.6 | 18.2 | 301 |
| | found 42.0 | 1.9 | 18.2 | |
| 7 | calc | | | 274-281 |
| | found | | | |
| 8 | calc 43.4 | 1.5 | 12.7 | 253 |
| | found 43.0 | 2.0 | 13.3 | |
| 9 | calc 34.9 | 2.1 | 11.1 | 300 |
| | found 35.0 | 2.4 | 11.0 | |
| 10 | calc 51.9 | 2.9 | 15.1 | 250 |
| | found 52.4 | 3.0 | 15.3 | |
| 11 | calc 49.1 | 2.7 | 14.3 | 240 |
| | found 49.0 | 3.0 | 14.3 | |
| 12 | calc 49.9 | 1.7 | 19.4 | 300 |
| | found 48.9 | 2.0 | 19.3 | |
| 13 | calc 39.7 | 1.2 | 12.6 | 300 |
| | found 39.5 | 1.6 | 12.5 | |
| 14 | calc 44.1 | 1.3 | 14.0 | 273 |
| | found 44.4 | 1.9 | 14.1 | |
| 15 | calc 41.8 | 1.3 | 13.3 | 300 |
| | found 41.4 | 1.7 | 13.4 | |
| 16 | calc 36.6 | 1.1 | 11.6 | 278 |
| | found 36.9 | 1.5 | 11.6 | |

| EXAMPLE NO. | ANALYSIS | | | MP/°C |
|---|---|---|---|---|
| | %C | %H | %N | |
| 17 | calc 39.7 | 1.2 | 12.6 | 294 |
| | found 39.8 | 1.5 | 12.4 | |
| 18 | calc 44.1 | 1.3 | 14.0 | 287 |
| | found 43.9 | 1.8 | 14.1 | |
| 19 | calc 31.3 | 1.0 | 10.0 | 300 |
| | found 31.7 | 1.4 | 10.1 | |
| 20 | calc 55.7 | 2.8 | 10.8 | 48 |
| | found 56.1 | 3.1 | 10.5 | |
| 21 | calc 38.6 | 3.6 | 19.3 | 75 |
| | found 38.9 | 3.7 | 19.2 | |
| 22 | calc | | | 249-253 |
| | found | | | |
| 23 | calc | | | 272-278 |
| | found | | | |
| 24 | calc 35.8 | 2.3 | 16.7 | 300 |
| | found 36.3 | 1.9 | 16.7 | |
| 25 | calc 35.5 | 1.0 | 13.8 | 300 |
| | found 36.1 | 1.4 | 13.8 | |
| 26 | calc 41.6 | 1.0 | 13.2 | 213-215 |
| | found 41.7 | 1.3 | 13.2 | |
| 27 | calc 41.6 | 1.0 | 13.2 | 300 |
| | found 42.0 | 1.4 | 13.4 | |
| 28 | calc 38.5 | 1.2 | 16.3 | 236 |
| | found 39.0 | 1.5 | 16.4 | |
| 29 | calc 40.4 | 1.2 | 17.1 | 236 |
| | found 39.8 | 1.4 | 16.8 | |
| 30 | calc 45.6 | 2.6 | 12.3 | 130 |
| | found 46.4 | 3.1 | 12.2 | |
| 31 | calc 38.6 | 2.0 | 10.4 | 131 |
| | found 39.0 | 2.4 | 10.2 | |

Examples 32 and 33 below describe the preparation of intermediates for the compounds of Example 20 and 21, respectively.

Example 32

Preparation of 3-chloro-4-cyano-5-(N-benzyl) isothiazole.

Benzylamine (1.1g) was added to a stirring solution of 3,5-dichloro-4-cyano isothiazole (0.9g) in tetrahydrofuran (50ml) at 20 °C. The mixture was stirred at 20 °C for 3 hours. The mixture was filtered and the filtrate evaporated. The residue was chromatographed using silica gel and chloroform as eluent to give the title compound as a white solid (1.2g, mp 164 °C).

| Analysis | | | |
|---|---|---|---|
| | %C | %H | %N |
| Calc.<br>Found | 52.9<br>53.3 | 3.2<br>3.3 | 16.8<br>16.8 |

Example 33

Preparation of 3-chloro-5-cyano-5-(N-ethoxymethyl) isothiazole.

Potassium carbonate (1.4g) and chloromethyl ethyl ether (2g) were added to a stirring solution of 3-chloro-4-cyano-5-amino isothiazole (1.6g) in acetonitrile (50ml). The mixture was stirred at 100 °C for 2 hours. The mixture was filtered and the filtrate evaporated. The residue was chromatographed using silica gel and dichloromethane as eluent to give the title compound as a white solid (1.0g, mp 200 °C).

| Analysis | | | |
|---|---|---|---|
| | %C | %H | %N |
| Calc.<br>Found | 38.6<br>38.9 | 3.6<br>3.7 | 19.3<br>19.2 |

Example 34

Pesticidal Activity

Pesticidal activity of compounds of the invention was assessed against larvae of the following pests:-

Spodoptera littoralis

Heliothis armigera

Plutella xylostella

The test methods employed for each species appear below. In each test, unless otherwise stated, solutions or suspensions of test compound were made up at a concentration in water of O.1%w containing 10%w acetone and 0.025%w "TRITON X-100" (trade mark) surface active agent (the condensation product of ethylene oxide with an alkyl phenol). These solutions were sprayed at a rate equivalent to 340 litres per hectare ($3.4 \times 10^{-5} m^3/m^2$) onto Petri dishes containing either test species per se or diet onto which test species were subsequently introduced, as indicated. In some assays leaf discs infested with test species were sprayed whilst other assays involved the spraying of plants which were infested subsequently with test species after the spray solution had dried. The tests were all conducted under normal insectary conditions (23 °C ± 2 °C, fluctuating humidity and light). Mortality assessments were made as indicated below, in terms of percentage mortality figures.

(i) Spodoptera littoralis (7 day) (S1 7D)

Test solutions were sprayed as indicated above onto Petri dishes containing a nutritious diet for Egyptian cotton leafworm larvae. When the spray deposit had dried, each dish was infested with ten 2nd instar larvae. Mortality assessments were made 7 days after spraying.

(ii) Spodoptera littoralis (foliar) (S1 Fol)

Test solutions were sprayed as described above onto Petri dishes containing 9cm discs of Chinese

cabbage leaves on filter papers. After drying, each dish was infested with ten 2nd instar larvae. Mortality assessments were made 24 hours after infestation.

(ii) Plutella xylostella (Px)

A test solution was sprayed as described above onto a 4 cm diameter Chinese cabbage leaf placed ventral side up in a pot. When the spray deposit had dried the pot was infested with 10 young instar larvae and a lid added. Mortality was assessed after 1 or 2 days, depending on when the test was carried out.

(iv) Heliothis armigera (Ha)

A test solution was sprayed as described above onto a 4 cm diameter Chinese cabbage leaf placed ventral side up in a pot. When the spray deposit had dried the pot was infested with 10 young instar larvae and a lid added. Mortality was assessed after 1 day.

Results of the assessments described in Examples 34 for each of the compounds of Examples 1 to 31 are provided in Table 3. In the Table grades A, B and C indicate mortalities of 70-100%, 40-69% and 0-39%, respectively, at the test concentration of O.1%w (1000 ppm). A blank space denotes that testing was not carried out.

**TABLE 3**

| Compound of Ex No. | Sl 7D | Sl Fol | Px | Ha |
|---|---|---|---|---|
| 1 | A | A | A | A |
| 2 | A | C | | |
| 3 | A | A | | |
| 4 | A | A | A | |
| 5 | A | A | | |
| 6 | A | | A | |
| 7 | A | A | B | |
| 8 | C | B | A | |
| 9 | A | A | A | |
| 10 | A | C | C | |
| 11 | C | A | C | |
| 12 | A | A | A | |
| 13 | A | A | | |
| 14 | A | A | A | |
| 15 | A | A | A | |
| 16 | A | A | A | A |
| 17 | A | A | A | A |
| 18 | A | A | A | |
| 19 | A | A | A | |
| 20 | B | A | A | |
| 21 | A | A | A | A |
| 22 | A | | | |
| 23 | A | | | |
| 24 | B | A | A | |
| 25 | C | A | C | C |
| 26 | A | A | A | |
| 27 | A | A | | |

| Compound of Ex No. | Sl 7D | Sl Fol | Px | Ha |
|---|---|---|---|---|
| 28 | A | A | | |
| 29 | A | A | B | |
| 30 | A | A | A | |
| 31 | A | A | A | |

Compounds of the present invention were also tested against other insect pests, including Aedes aegypti (yellow fever mosquito), Musca domestica (housefly), Acyrthosiphon pisum (pea aphid), Megoura viciae (vetch aphid), Phaedon cochleariae (mustard beetle), Trialeurodes vaporariorum (greenhouse whitefly), Nephotettix cincticeps (green leaf hopper) and Nilaparvata lugens (brown rice plant hopper). However, in most cases this revealed no or low activity. Preliminary conclusions are that the activity of the compounds found, in the majority of tests, against caterpillar pests, is surprising, given the otherwise unpromising insecticidal activity of the compounds.

Further glasshouse tests have shown particularly interesting activity against larval pests from the compounds of Examples 1, 7, 9, 13, 14, 15, 16, 17, 18 and 19.

## Claims

1. A method of combating caterpillar pests at a locus, which method comprises treating the locus with a compound of general formula

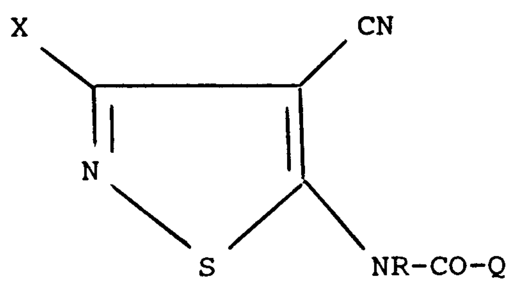

I

where X represents a halogen atom, R represents a hydrogen atom or an optionally substituted alkyl group, and Q represents an optionally substituted aryl or heteroaryl group.

2. A method as claimed in claim 1, wherein R represents a hydrogen atom or a benzyl or ($C_{1-4}$ alkoxy)-methyl group.

3. A method as claimed in claim 2, wherein R represents a hydrogen atom.

4. A method as claimed in any preceding claim, wherein X represents a chlorine atom.

5. A method as claimed in any preceding claim, wherein Q represents an optionally substituted phenyl group.

6. A method as claimed in claim 5, wherein Q represents a phenyl group having a halogen substituent at the 3-position, and being optionally substituted elsewhere in the ring.

7. A method as claimed in claim 6, wherein Q represents a 3-halophenyl, 3,4-dihalophenyl or 3,5-dihalophenyl group.

**8.** Use as a larvicide of a compound of general formula I, as defined in any preceding claim.

**9.** A compound of general formula I, as claimed in claim 1, wherein R represents an optionally substituted alkyl group.

**10.** A compound of general formula I, as defined by claim 6 or 7.

**11.** A process for the preparation of a compound of general formula I as claimed in claim 9 or claim 10, which comprises reacting together compounds of general formula II and III

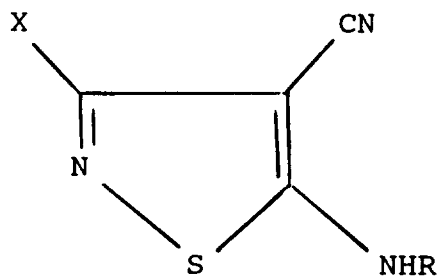

II

Hal-CO-Q III

**12.** A compound of general formula I, as claimed in claim 9 or 10, when prepared by a process as claimed in claim 11.

**13.** A pesticidal composition comprising a carrier and, as active ingredient, a compound of general formula I as claimed in any of claims 9, 10 or 12.

**14.** A method, use, compound, composition or process in accordance with the invention, substantially as hereinbefore described with particular reference to the Examples.

European Patent Office

# EUROPEAN SEARCH REPORT

Application Number
EP 94 20 1225

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A<br>D | DE-A-15 95 992 (E.MERCK AG)<br>& US-A-3 541 108 (E.MERCK AG)<br>--- | | A01N43/80<br>C07D275/03 |
| A<br><br>D | FR-A-2 014 527 (E.MERCK AKTIENGESELLSCHAFT)<br>& GB-A-1 226 913 (E.MERCK AKTIENGESELLSCHAFT)<br>--- | | |
| A | CHEMICAL PATENTS INDEX, DOCUMENTATION ABSTRACTS JOURNAL<br>Section Ch, Week 8936, 1 November 1989<br>Derwent Publications Ltd., London, GB;<br>Class C, AN 260915<br>& JP-A-1 190 670 (NISSAN CHEM IND KK) 31 July 1989<br>----- | | |

| TECHNICAL FIELDS SEARCHED (Int.Cl.5) |
|---|
| A01N<br>C07D |

The present search report has been drawn up for all claims

| Place of search | Date of completion of the search | Examiner |
|---|---|---|
| THE HAGUE | 8 August 1994 | Donovan, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)